## Europäisches Patentamt
## European Patent Office
## Office européen des brevets

(19)

(11) Numéro de publication: **0 180 496**
**B1**

# FASCICULE DE BREVET EUROPÉEN

(12)

(45) Date de publication du fascicule du brevet:
**31.05.89**

(21) Numéro de dépôt: **85401902.3**

(22) Date de dépôt: **30.09.85**

(51) Int. Cl.⁴: **C 07 K 15/00,** C 07 K 3/02,
C 12 P 21/00, C 12 N 15/00,
A 61 K 37/02, A 61 K 39/395,
G 01 N 33/574, G 01 N 33/577 //
(C12P21/00, C12R1:91)

(54) Protéines de differenciation acino-foetales associées au cancer du pancréas, antisérum et anticorps monoclonaux contre ces proteines, procédés de préparation.

(30) Priorité: **01.10.84 FR 8415081**

(43) Date de publication de la demande:
**07.05.86 Bulletin 86/19**

(45) Mention de la délivrance du brevet:
**31.05.89 Bulletin 89/22**

(84) Etats contractants désignés:
**AT BE CH DE FR GB IT LI LU NL SE**

(73) Titulaire: **CENTRE NATIONAL DE LA RECHERCHE SCIENTIFIQUE (CNRS), 15, Quai Anatole France, F-75007 Paris (FR)**

(72) Inventeur: **Escribano Crespo, Maria Juana, 6, Allée du Prunier Hardy, F-92220 Bagneux (FR)**
Inventeur: **Burtin, Pierre, 28, rue des Cévennes, F-75015 Paris (FR)**

(74) Mandataire: **Warcoin, Jacques, Cabinet Régimbeau 26, avenue Kléber, F-75116 Paris (FR)**

(56) Documents cité:
SCANDINAVIAN JOURNAL OF GASTROENTEROLOGY, vol. 16, no. 8, 1981, pages 1033-1039; W.-H. SCHMIEGEL et al.: "Pancreatic oncofetal antigen in pancreatic juices. Partial chemical characterization and diagnostic application of a pancreatic cancer-associated antigen"
SCANDINAVIAN JOURNAL OF IMMUNOLOGY, vol. 8, suppl. 8, 1978, pages 471-478, Blackwell Scientific Publications; S.R. HARVEY et al.: "An immunoglobulin M(IgM) antibody to carcinoembryonic antigen (CEA)-line antigen in human pancreatic cancer"
PROTIDES BIOLOGICAL FLUIDS, vol. 24, 1976, pages 529-533; R. FRITSCHE et al.: "Beta-oncofoetal antigen (BOFA) associated with several types of human cancers"
CHEMICAL ABSTRACTS, vol. 96, no. 7, 15 février 1982, page 290, no. 48642v, Columbus, Ohio, US
CHEMICAL ABSTRACTS, vol. 91, no. 21, 19 novembre 1979, page 479, no. 173027w, Columbus, Ohio, US; K. NISHIDA et al.: "Assay of pancreatic carcinoembryonic antigen in pancreatic juice for the diagnosis of pancreatic cancer"

(56) Documents cité: (suite)
ANNALS OF THE NEW YORK ACADEMY OF SCIENCES, vol. 259, 1975, pages 377-381; T. YAMAMOTO et al.: "Heterogeneity of carcinoembryonic antigen. II. Monosaccharide composition of concanavalin a-reactive and -nonreactive CEA"
GASTROENTEROLOGIA JAPONICA, vol. 16, no. 3, 1981, pages 268-274, The Japanes Society of Gastroenterology, JP; T. HOMMA et al.: "A pancreatic oncofetal antigen: its partial purification and clinical application"
CHEMICAL ABSTRACTS, vol. 98, no. 17, 25 avril 1983, page 434, no. 141650x, Columbus, Ohio, US; T. HOMMA: "Pancreatic oncofetal antigen (POA). Its characterization and clinical detection by enzyme immunoassay"

Le dossier contient des informations techniques présentées postérieurement au dépôt de la demande et ne figurant pas dans le présent fascicule.

EP 0 180 496 B1

**Description**

La présente invention concerne des protéines de différenciation acino-foetales associées au cancer du pancréas, leur purification, un antisérum spécifique, et sa prépatarion, des anticorps monoclonaux spécifiques de ces protéines et leur procédé de préparation ainsi que les compositions à usage diagnostique et à usage thérapeutique les contenant.

La mise en évidence de la présence, dans des extraits de pancréas foetal, d'antigènes analogues à ceux des cellules pancréatiques tumorales est connue.

Des antigènes pancréatiques oncofoetaux humains ont été identifiés, ayant pour poids moléculaire 40 KD (POA-1) (banwo O., Versey J. et Hobbs J.R., New Oncofetal Antigen for Human·Pancreas. Lancet, 1: 643 - 645, 1974; Hobbs J.R., Knapp, M.L. et Branfoot A.G., Pancreatic Oncofetal Antigen (POA): its frequency and Localisation in Humans. Oncod. Biol. and Medicine, 1: 37 - 48, 1980), 800 et 900 KD (POA-2) (Gelder F.B., Resse C.J., Moossa A.R., Hall T. et Hunter R., Purification partial characterization and clinical evaluation of a pancreatic oncofetal antigen. Cancer Res. 38: 313 - 324, 1978) et 1 000 KD (PCAA) (Shimano T., Loor R.M. Papsidero L.D. et al., Isolation, characterization and clinical evaluation of a pancreas cancer associated antigen, Cancer., Supplement, 47: 1602 - 1620, 1881) POA in pancréatic juices, search J. Gastroenterol. 16/8, 1981, pages 1033 - 1039, et Heterogenety of catcinoembryonic antigen, Ann. N.Y. Acad. Sci. 259 1975, pages 377 - 351. Cependant aucun de ces antigènes n'est spécifique du cancer du pancréas car ils se retrouvent dans des tissus différents des tumeurs cancéreuses pancréatiques.

La présente invention concerne des protéines de différenciation acino-foetales associées au cancer du pancréas, caractérisées en ce qu'il s'agit de glycoprotéines manosidées, de masse moléculaire apparente moyenne choisie parmi: 120 KD, 94 KD, 75 KD et 58 KD.

La présente invention concerne également un procédé de préparation de ces protéines, caractérisé en ce qu'on prépare un homogénat de pancréas foetal humain soluble et que l'on isole une fraction constituée par des glycoprotéines manosidées.

Le procédé de préparation de ces antigènes comporte les étapes suivantes:

a) on prépare un homogénat de pancréas foetal humain soluble,

b) on traite l'homogénat par chromatographie sur colonne d'affinité sur concanavaline A couplée à un gel afin de fixer lesdites protéines,

c) on élue les protéines en cause, et

d) éventuellement, on purifie les protéines obtenues.

La préparation de l'homogénat est décrite à l'exemple 1.

On utilise des foetus jeunes de moins de 6 mois de gestation, de préférence de moins de 5 mois, parce que les protéines selon l'invention n'apparaissent pas dans le pancréas adulte et que des lapins injectés avec du pancréas de foetus âgés de plus de 6 mois n'ont pas produit d'anticorps décelables.

Dans un mode particulier de réalisation, on à utilisé du gel de Sépharose 4B couplé à la concanavaline A, commercialisé par Pharmacia. La fraction non fixée est éliminée, la fraction fixée est éluée, à l'aide d'un ligand spécifique, par ex., l'α-méthylmanoside 0,2 M. On dyalise alors la fraction fixée éluée par une technique classique et on sépare les protéines par exemple par électrophorèse préparative. On à préalablement repéré les pics correspondant aux protéines selon l'invention par électrophorèse qualitative.

Les fractions de gel d'acrylamide chargées en protéines selon l'invention sont alors prélevées, et les protéines en sont extraites par électroélution dans un boyau de dialyse contre un tampon au phosphate P.B.S. (solution saline 0,15 M NaCl tamponnée à pH 7 avec tampon phosphate de potassium 0,01 M final). On dialyse contre de l'eau distillée et on lyophilise. Une caractérisation des protéines selon l'invention est décrite à l'exemple 6.

Pour séparer les protéines de différenciation, on peut également utiliser les techniques d'imunofixation sur les anticorps monoclonaux.

La présente invention concerne également un antisérum contre les antigènes selon l'invention.

Par la technique de "blotting" (après électrophorèse en gel d'acrylamide - PAGE -, on transfert électrophorétiquement les protéines ayant migré dans le gel à une feuille de nitrocellulose; cette feuille est ensuite traitée comme dans la technique du NIF.), cet antisérum reconnaît, dans les extraits de pancréas foetal, 4 constituants de masse moléculaire apparente moyenne 120, 94, 75 et 58 KD (à 10 % près).

La présente invention concerne également un procédé de préparation de cet antisérum comportant les étapes suivantes:

a) on injecte à un animal un extrait de pancréas foetal humain de moins de 6 mois,

b) on saigne l'animal et on récupère un antisérum,

c) on décomplémente cet antisérum,

d) on absorbe l'antisérum décomplémenté,

e) on centrifuge l'antisérum décomplémenté absorbé, et

f) on récupère l'antisérum spécifique.

De préférence, on injecte à l'animal un extrait de pancréas foetal de moins de 5 mois.

On prépare cet antisérum selon le protocole décrit à l'exemple 2 contre des extraits pancréatiques bruts préparés comme le décrit l'exemple 1. Dans le mode particulier de réalisation décrit à l'exemple 1, seules les saignées n° 4 et 5 sont positives dans un test de présence d'anticorps.

Dans un mode de réalisation de l'invention, on à injecté l'extrait à un lapin. Les souris et les poules, par

EP 0 180 496 B1

exemple, peuvent également produire un antisérum contre ces extraits.

Pour décomplémenter l'antisérum, on chauffe au bainmarie à environ 56°C pendant 1/2 heure, selon une technique classique. On rend ensuite l'antisérum spécifique du pancréas foetal par absorption de différents extraits, par exemple des extraits de pancréas adulte normal, du sérum normal, des globules rouges A, O, B, et éventuellement d'autres organes.

Pour ceci, on ajoute 2 ml de sérum normal par ml d'antisérum et 250 mg d'extraits de pancréas adulte normal lyophilisé par ml d'antisérum.

Pour obtenir l'extrait de pancréas adulte normal, on procède comme à l'exemple 1, le pancréas provient de donneur jeunes morts accidentellement.

On purifie l'antisérum décomplémenté absorbé par une technique classique de centrifugation.

On à testé l'antisérum absorbé purifié pour sa faculté à déceler les antigènes par deux techniques:

### A) Immunofixation sur membranes de nitrocellulose (technique NIF)

Cette technique est décrite à l'exemple 3. Elle permet de déceler de très faibles quantités d'un ou plusieurs antigènes dans les mélanges complexes, par exemple des extraits bruts d'organes, des sérums pathologiques. Cette technique permet de déceler approximativement jusqu'à 0,1 µg de protéines de différenciation par ml dans les extraits bruts d'organes ou les liquides biologiques.

La sensibilité varie avec le système antigène-anticorps et peut aller jusqu'à 0,01 µg d'antigènes par ml, soit 0,0005 % lorsque, comme pour les tests rassemblés dans le tableau 1, les extraits sont analysés à partir de 20 mg/ml.

Par cette technique, l'antisérum s'est révélé positif pour les extraits de pancrés foetal, les tumeurs de pancréas et des métastases hépatiques d'un cancer primitif du pancréas. Par contre, ont été révélés négatifs, d'autres extraits d'organes adultes ou foetaux détaillés dans le tableau I.

### Tableau I
#### Extraits d'organes normaux et serum normal

| Organes | Foetal | adulte |
|---|---|---|
| Pancréas | + | - |
| Foie | - | - |
| Estomac | - | - |
| Poumon | - | - |
| Intestin | - | - |
| Rein | - | - |
| Rate | - | - |
| Serum normal | - | - |

. Les extraits sont des "pools" d'organes utilisés à concentration décroissante à partir de 20 mg/ml.
. N.T. = Non Testé

Par cette même technique, 100 % des sérums de malades atteints de cancers du pancréas ont été positifs (N = 17) alors que le sérum de patients ayant d'autres tumeurs était négatif (voir tableau II).

### Tableau II
#### Serums pathologiques

| Serums pathologiques | Nombre testés | Nombre positifs |
|---|---|---|
| Cancer pancréas | 17 | 17 |
| Cancer estomac | 2 | 0 |
| Cancer colon | 2 | 0 |
| Cancer poumon | 2 | 0 |
| Cancer rein | 2 | 0 |

. Les sérums sont utilisés à des dilutions 1g2 à partir de la concentration normale.

### B) Immunohistologie sur coupes fixées à l'éthanol ou au liguide de Bouin

3

La méthode utilisée est décrite à l'exemple 4.

En utilisant soit la technique d'immunofluorescence, soit l'immunopéroxydase indirecte, l'antisérum marque les cellules acineuses foetales jusqu'à environ le 6ème mois de la gestation et est négatif pour d'autres organes foetaux ou adultes. Il à marqué tous les cancers du pancréas analysés et à été négatif pour une large variété d'autres types de cancer. Les résultats de l'immunohistologie sont détaillés dans le tableau III.

**Tableau III**

Réaction de l'antisérum anti-pancréas foetal en NIF et immunohistologie.

| Cancer | Nombre testés | Positifs | Négatifs |
|---|---|---|---|
| Pancréas | 18 | 18 | 0 |
| Foie | 11 | 0 | 11 |
| Estomac | 8 | 0 | 8 |
| Colon | 10 | 0 | 10 |
| Glandes salivaires | 4 | 0 | 4 |
| Poumon | 4 | 0 | 4 |
| Vessie | 6 | 0 | 6 |
| Vésicule biliaire | 6 | 0 | 6 |
| Sein | 3 | 0 | 3 |
| Prostate | 7 | 0 | 7 |
| **Tissus normaux** | | | |
| Pancréas foetal 6 mois et moins | 20 | 18 | 2 ($\pm$) |
| Pancréas foetal plus de 6 mois | 16 | 8 | 8 |
| Pancréas adulte | 6 | 0 | 6 |
| Duodénum | 4 | 0 | 4 |
| Estomac | 2 | 0 | 2 |
| Vésicule biliaire (canaux) | 5 | 0 | 0 |
| Glandes salivaires | 3 | 0 | 3 |
| Pancréatite chronique | 11 | 2 | 9 |

Les pancréatites chroniques constituent un cas à part car elles sont en général négatives, mais dans certains cas on observe un marquage similaire à celui trouvé dans les zones péritumorales.

L'antisérum selon l'invention permet donc de déceler des taux sériques et tissulaires trop faibles pour être dosés par précipitation dont le seuil de sensibilité se situe autour de 10 µg/ml.

La Demanderesse à mis en évidence que les protéines selon l'invention apparaissent non seulement dans les tumeurs pancréatiques, mais encore dans les lésions prétumorales. Ces protéines de différenciation ont donc en outre valeur de margueurs prétumoraux.

En effet, au cours de la cancérogénèse chimique expérimentale chez le hamster, ils sont exprimés vers le deuxième mois alors que les premières tumeurs ne sont décelées à l'histologie que vers le 8ème mois.

Chez l'homme, ces antigènes l'expriment intensément non seulement dans les cancers mais encore dans les marqueurs prétumoraux.

Le tableau suivant présente d'autres résultats d'analyse montrant la spécificité de ces protéines de différenciation pour d'autres tissus normaux foetaux et adultes, par les techniques du NIF et d'immunohistologie.

**Tableau IV**

| Tissus | Foetal | | Adulte | |
|---|---|---|---|---|
| | NIF | Hist | NIF | Hist |
| Pancréas | + | + | - | - |
| Foie | - | - | - | - |
| Poumon | - | - | - | - |
| Estomac | - | - | - | - |
| Colon | - | - | - | - |
| Intestin grêle | - | - | - | - |

| | | | |
|---|---|---|---|
| Glandes salivaires | NT | NT | NT | - |
| Vessie | " | - | - | - |
| Vésicule biliaire | " | - | " | - |
| Muscle | - | - | " | - |
| Peau | - | NT | - | NT |
| Cerveau | - | " | - | " |
| Rate | - | - | - | - |
| Rein | - | - | - | - |
| Serum | - | | - | - |

+ réaction positive; - réaction négative; NT non testé

Ces protéines de différenciation selon l'invention peuvent également être appliquées à l'élaboration d'anticorps spécifiques.

Ainsi, la Demanderesse à réalisé la préparation anticorps monoclonaux spécifique des protéines de différenciation selon l'invention.

C'est pourquoi, la présente invention concerne des anticorps monoclonaux spécifique des protéines de différenciation de masse moléculaire apparente 120 KD, 94 KD, 75 KD et 58 KD.

La présente invention concerne également un procédé de préparation de ces anticorps monoclonaux, caractérisé en ce qu'il comporte les étapes suivantes:

a) on immunise une souris avec au moins une des protéines de différenciation,

b) on réalise une fusion cellulaire de cellules de rate de ces souris, avec un myélome $SP_2$,

c) on cultive les hybridomes obtenus dans un milieu approprié,

d) on sélectionne les cultures par criblage,

e) on conserve et on clone les hybridomes positifs au criblage spécifiquement avec le pancréas foetal, et,

f) on isole et éventuellement on purifie l'anticorps monoclonal.

Pour préparer les anticorps, on utilise donc la technique classique de l'hybridation, telle que mise au point par Kohler et Milstein en 1975, en immunisant des souris, par exemple des souris BALB/C avec la fraction d'un homogénat de pancréas foetal de moins de 6 mois purifiée sur Concanavaline A préalablement préparée. Dans un mode particulier de réalisation de l'invention, on à utilisé pour immuniser les souris, des protéines de différenciation préparées selon l'invention à partir de pancréas foetaux âgés de 3 à 4 mois.

Pour le criblage, c'est-à-dire la sélection des hybridomes producteurs, on utilise par exemple la technique du NIF, déjà décrits. On teste les cultures avec divers extraits, par exemple des extraits de pancréas adulte normal, du sérum adulte normal et foetal, et d'un extrait de pancréas foetal, pour ne conserver que les cultures qui réagissent spécifiquement avec le pancréas foetal.

Les anticorps monoclonaux sont alors sélectionnés par des techniques classiques. On peut ainsi utiliser la technique "Immuno Blot" mettant en oeuvre du SDS-PAGE (exemple 5), technique également connue sous le nom de "Western Blot" et décrite par Towbin et al. (Electrophoretic Transfer of Proteins from Polyacrylamides Gels to Nitrocellular Sheets Procedure and Some Application, Prot. Nat. Acad. Sci. USA 76, 1979 page 350 - 354).

On isole alors les anticorps monoclonaux dans le surnageant des cultures d'hybridomes sélectionnées, par une technique usuelle.

On peut aussi obtenir des anticorps monoclonaux à partir des ascites en injectant à des souris la cellule productrice de l'anticorps monoclonal.

La Demanderesse a ainsi isolé deux anticorps monoclonaux spécifiques de la protéine de différenciation selon l'invention de masse moléculaire moyenne 120 KD.

Par typage, on met en évidence qu'il s'agit d'anticorps de type Ig G.

On peut alors isoler les anticorps par chromatographie d'affinité sur protéine A fixée sur sépharose, par exemple, en éluant à un pH compris entre 3 et 6.

La présente invention concerne donc également des compositions à usage diagnostique contenant à titre d'élément actif au moins une protéine de différenciation ou un anticorps monoclonal selon l'invention et un substrat acceptable, par exemple sous forme de kits d'immunodiagnostic selon un mécanisme basé sur des réactions anticorps-antigène.

Les protéines de différenciation et les anticorps monoclonaux selon l'invention sont également utiles en tant que marqueurs spécifiques tumoraux et prétumoraux pouvant être utilisés pour l'imagerie.

C'est pourquoi la présente invention concerne les compositions à usage diagnostique comportant les protéines de différenciation ou les anticorps monoclonaux selon l'invention marqués.

Pour cette utilisation, le marquage peut être réalisé soit par tout marqueur, en particulier les marqueurs fluorescent, radioactif ou paramagnétique.

Enfin, la présente invention concerne également les compositions à usage thérapeutique comportant au moins une protéine de différenciation ou un anticorps monoclonal selon l'invention conjugué à un principe actif.

**Exemple 1**

Préparation d'extraits de pancréas foetal

On prélève sur des foetus en bon état, après avortement thérapeutique, à 3 à 4 mois de gestation, le pancréas qui est rapidement plongé dans une solution d'antiprotéase contenant de l'acide aminocaproïque (0,4 %) et de l'aprotinine (40 unités de Kallikreine/ml), par exemple l'aprotinine Trasylol® commercialisée par Sigma.

Le pancréas, à raison de 100 mg/ml de solution d'antiprotéase, est broyé le plus tôt possible après le prélèvement avec un broyeur Ultraturax pendant quelques minutes à froid.

L'extrait est centrifugé à 20 000 tours/minute pendant 1/2 heure, le surnageant est aliquoté et conservé à -80°C.

Ces extraits contiennent entre 5 et 8 mg de protéines par ml.

**Exemple 2**

Préparation de l'antisérum

L'extrait de pancréas ci-dessus décrit est injecté à un lapin selon le protocole suivant:

Jour 1 : 1 ml d'extrait + 1 ml d'adjuvant complet de Freund en injections intradermiques sur plusieurs points au dos.
. Jour 24 : Même opération que Jour 1.
. Jour 44 : Saignée d'essai n° 1.
. Jour 54 : Re-immunisation comme Jours 1 et 24.
. Jour 78 : Saignée d'essai n° 2.
. Jour 98 : Nouvelle injection comme précédemment.
. Jour 110: Saignée d'essai n° 3.
. Jour 150: Injection intraveineuse sans adjuvant (solution bien claire centrifugée).
. Jour 156: Saignée d'essai n° 4.
. Jour 186: Nouvelle injection intraveineuse.
. Jour 192: Saignée n° 5.

Le sérum est alors décomplémenté, en le portant au bain-marie à 56°C pendant 30 minutes environ, puis absorbé par des extraits de pancréas adulte normal, du sérum normal et des globules rouges A, O, B.

**Exemple 3**

Technique de NIF

. Découper une pièce de nitrocellulose de dimensions appropriées (membranes de nitrocellulose BA85, Schleicher et Schüll - Dassel, R.D.A.).

. Déposer la solution dans laquelle l'antigène est recherché en microgouttes de 1 à 5 µl. La concentration est choisie en fonction de la sensibilité désirée. Les dilutions des antisérums sont faites dans une solution à 2 % d'ovalbumine de blanc d'oeuf, dans l'eau distillée, commercialisée par Sigma.

. Laisser sécher. Rincer avec du P.B.S.

. Incuber 1 heure à 4°C dans une solution de protéine saturante (solution à 2 % d'ovalbumine de blanc d'oeuf, dans l'eau distillée, commercialisée par Sigma) pour bloquer les sites de fixation de la membrane non occupée par l'antigène.

. Rincer avec du P.B.S. Incuber avec l'antisérum. Dans ce cas, pour le sérum anti-pancréas foetal absorbé, on l'utilise dilué 1/1000 pendant une nuit à 4°C.

. Laver abondamment le filtre pour éliminer complètement l'antisérum.

. Incuber avec l'anticorps anti IgG de lapin conjuguée à la péroxydase (Institut Pasteur Production), dilué 1/2000 pendant 2 heures à 4°C.

. Laver à nouveau abondamment avec du P.B.S.

. Révéler la réaction enzymatique par incubation dans le substrat de la péroxydase: $H_2O_2$ 0,01 % et le chromogène. Celui-ci peut être soit le chloronaphtol (Sigma): coloration bleue, soit le 3,3'-diaminobenzidine (D.A.B., Merck): coloration marron.

Le premier est dissous à 3 mg/ml dans le méthanol puis dilué 5 fois dans le P.B.S. Le deuxième (D.A.B.) est dissous directement dans le P.B.S. (0,5 mg/ml). Dans les deux cas, ajouter du péroxyde d'hydrogène a concentration finale de 0,01 % comme indiqué plus haut.

. Lorsque la réaction est développée (stabilisation de la couleur), laver à l'eau distillée et pour le D.A.B., fixer la couleur par lavage au HCl $10^{-2}$ M.

**Exemple 4**

Immunohistologie

Les coupes d'organes, fixées soit à l'éthanol, soit au formol, soit encore au liquide de Bouin, sont incluses dans la paraffine. Déparaffiner, hydrater et inhiber la péroxydase endogène.

1) Incuber avec de la sérumalbumine bovine (B.S.A.) à 2 % dans de l'eau distillée, 1 heure à 4°C.

2) Incuber avec de l'antisérum anti-pancréas foetal dilué 1/25 dans B.S.A. 1 %, 4 heures, à 4°C.

3) Laver 3 fois 10 minutes dans P.B.S.

4) Incuber avec les anticorps anti IgG de lapin conjugués à la péroxydase (Institut Pasteur Production) dilués 1/100 dans du P.B.S.

5) Laver 3 fois 10 minutes dans P.B.S.

6) Révéler la réaction enzymatique dans un bain de $H_2O_2$ 0,01 % et amino-éthylcarbazole.

**Exemple 5**

Immunoanalyse sur tâches de nitrocellulose après SDS-électrophorèse sur gel d'acrylamide.

On met en oeuvre la technique SDS-PAGE dans des gels à 10 % d'acrylamide dans un tampon Tris-glycine pH 8,3 contenant 0,1 % de SDS (Laenmli U.K. Cleavage and Structural Protéins during Assembly of Head of the Bacteriophage Nature (Lond. 227: 680 - 5 (1970)).

On transfert aux membranes de nitrocellulose dans une cellule de transfert Biorad à 4° C sous 100 mA en courant constant pendant 16 h à pH 8,3 (Towbin). Les tâches sont révélées comme dans la technique du NIF.

**Exemple 6**

Caractérisation des protéines de différenciation spécifique des pancréas par la technique des immunoblots après électrophorèse sur gel acrylamide.

Des homogénats de pancrés adultes tumoraux et foetaux (17, 20, 25 et 32 semaines) sont soumis à une analyse par SDS-PAGE et sont colorés avec du bleu de Coomassie ou transférés aux membranes de nitrocellulose.

Plus tard, le sérum anti-pancréas foetal à révélé plusieurs bandes correspondant aux différentes protéines de différenciation.

D'après l'intensité de la coloration, la plupart des protéines sont presque absentes dans les pancréas de 32 semaines.

Les pancréas adultes n'ont pas été colorés.

Dans une tumeur, deux protéines sont présentes, alors que dans une autre est exprimée principalement la protéine de masse moléculaire 120 K.

L'évaluation de l'intensité de la coloration par rapport à la référence indique que dans tous les cas, les protéines de différenciation sont présentes en faible quantité par rapport à la quantité totale de protéines dans les homogénats bruts.

**Revendications**

1. Protéines de différenciation acino-foetales associées au cancer du pancréas, caractérisées en ce qu'il s'agit de glycoprotéines manosidées, de masse moléculaire apparente moyenne choisie parmi: 120 KD, 94 KD, 75 KD et 58 KD, et provenant notamment du pancréas foetal humain âgé de moins de 6 mois de gestation.

2. Procédé de préparation de protéines selon la revendication 1, caractérisé en ce que l'on prépare un homogénat de pancréas foetal humain soluble et que l'on isole une fraction constituée par des glycoprotéines manosidées.

3. Procédé de préparation de protéines de différenciation acino-foetales associées au cancer du pancréas à partir d'extraits pancréatiques foetaux humains selon la revendication 2, comportant les étapes suivantes:

a) on prépare un homogénat de pancréas foetal humain soluble,

b) on traite l'homogénat par chromatographie sur colonne d'affinité sur concanavaline A couplée à un gel afin de fixer lesdites protéines,

c) on élue les protéines en cause, et

d) éventuellement, on purifie les protéines obtenues.

4. Procédé selon la revendication 2 ou 3, caractérisé en ce que le pancréas foetal humain est âgé de moins de 6 mois, de préférence moins de 5 mois.

5. Antisérum polyclonal contre les protéines selon la revendication 1, caractérisé en ce qu'il est spécifique du cancer pancréatique.

6. Procédé de préparation d'un antisérum selon la revendication 5, comportant les étapes suivantes:
a) on injecte à un animal un extrait de pancréas foetal humain de moins de 6 mois,
b) on saigne l'animal et on récupère un antisérum,
c) on décomplémente cet antisérum,
d) on absorbe l'antisérum décomplémenté,
e) on centrifuge l'antisérum décomplémenté absorbé, et
f) on récupère l'antisérum spécifique.

7. Procédé de préparation d'un antisérum selon la revendication 5, caractérisé en ce qu'on injecte un extrait de pancréas foetal humain du foetus âgé de moins de 5 mois.

8. Procédé de préparation d'un antisérum selon la revendication 6 ou 7, caractérisé en ce qu'on absorbe l'antisérum, en particulier sur globules rouges A, O, B, le sérum normal et/ou du pancréas normal.

9. Anticorps monoclonal spécifique d'une protéine selon la revendication 1.

10. Procédé de préparation d'un anticorps monoclonal spécifique d'une protéine selon la revendication 1, caractérisé en ce qu'il comporte les étapes suivantes:
a) on immunise une souris avec au moins une des protéines de différenciation,
b) on réalise une fusion cellulaire de cellules de rate de ces souris, avec un myélome $SP_2$,
c) on cultive les hybridomes obtenus dans un milieu approprié,
d) on sélectionne les cultures par criblage,
e) on conserve et on clone les hybridomes positifs au criblage spécifiquement avec le pancréas foetal, et,
f) on isole et éventuellement on purifie l'anticorps monoclonal.

11. Procédé selon la revendication 10, caractérisé en ce que les protéines de différenciation proviennent d'un foetus âgé de 3 à 4 mois.

12. Composition à usage diagnostique contenant à titre d'élément actif au moins une protéine de différenciation ou un anticorps monoclonal selon l'une des revendications 1 ou 9 et un substrat acceptable.

13. Composition selon la revendication 12, caractérisée en ce que la protéine de différenciation ou l'anticorps est marqué.

14. Composition selon la revendication 12 ou 13, caractérisé en ce qu'il s'agit d'un marquage radioactif, paramagnétique ou fluorescent.

15. Composition à usage thérapeutique comportant au moins une protéine de différenciation selon la revendication 1 ou un anticorps monoclonal selon la revendication 9 conjugué à un principe actif.


**Patentansprüche**

1. Azino-fötale Differenziationsproteine, die den Pancreas-Krebs begleiten, dadurch gekennzeichnet, daß es sich dabei um manosidische Glycoproteine handelt mit einem scheinbaren mittleren Molekulargewicht ausgewählt aus 120 KD, 94 KD, 75 KD und 58 KD, und insbesondere vorkommen im fötalen humanen Pancreas mit einem Alter von weniger als sechs Schwangerschaftsmonaten.

2. Verfahren zur Herstellung von Proteinen nach Anspruch 1, dadurch gekennzeichnet, daß man ein lösliches Homogenat von fötalem humanem Pancreas herstellt und daraus eine Fraktion isoliert, die sich aus den monosidischen Glycoproteinen zusammensetzt.

3. Verfahren zur Herstellung von Azino-fötalen Differenziationsproteinen, die den Pacreas-Krebs begleiten, ausgehend von fötalen humanen Pancreas-Extrakten nach Anspruch 2, das die folgenden Stufen umfaßt:
a) man stellt ein lösliches Homogenat aus fötalem humanem Pancreas her,
b) man behandelt das Homogenat durch Chromatographie auf einer Affinitätssäule auf Concanavalin A, gekuppelt an ein Gel, um die genannten Proteine zu binden,
c) man eluiert die fraglichen Proteine, und
d) gegebenenfalls reinigt man die erhaltenen Proteine.

4. Verfahren nach Anspruch 2 oder 3, dadurch gekennzeichnet, daß der fötale humane Pancreas weniger als sechs Monate alt ist, bevorzugt weniger als fünf Monate.

5. Polyclonales Antiserum gegen die Proteine nach Anspruch 1, dadurch gekennzeichnet, daß es spezifisch für Pancreas-Krebs ist.

6. Verfahren zur Herstellung eines Antiserums nach Anspruch 5, das die folgenden Stufen umfaßt:
a) man injiziert einem Tier einen Extrakt des humanen fötalen Pancreas von weniger als sechs Monaten,
b) man tötet das Tier und man gewinnt ein Antiserum,
c) man dekomplementiert dieses Antiserum,
d) man absorbiert das dekomplementierte Antiserum,
e) man zentrifugiert das absorbierte dekomplementierte Antiserum, und
f) man gewinnt das spezifische Antiserum.

7. Verfahren zur Herstellung eines Antierums nach Anspruch 5, dadurch gekennzeichnet, daß man einen Extrakt aus einem fötalen humanen Pancreas von einem Fötus von weniger als fünf Monaten extrahiert.

8. Verfahren zur Herstellung eines Antiserums nach Anspruch 6 oder 7, dadurch gekennzeichnet, daß man das Antiserum absorbiert, insbesondere auf roten Blutkörperchen A, O, B, Normalserum und/oder normalem Pancreas.

8

9. Monoclonaler Antikörper, spezifisch für ein Protein nach Anspruch 1.

10. Verfahren zur Herstellung eines monoclonalen Antikörpers, spezifisch für ein Protein nach Anspruch 1, dadurch gekennzeichnet, daß es die folgenden Stufen umfaßt:

a) man immunisiert eine Maus mit wenigstens einem der Differenziationsproteine,

b) man führt eine Zellverschmelzung von Milzzellen dieser Mäuse mit einem Myelom SP$_2$ durch,

c) man kultiviert die erhaltenen Hybridome in einem geeigneten Milieu,

d) man selektioniert die Kulturen durch Auswählen,

e) man konserviert und man clont die Auswahl-positiven Hybridome spezifisch mit dem fötalen Pancreas, und

f) man isoliert und reinigt gegebenenfalls den monoclonalen Antikörper.

11. Verfahren nach Anspruch 10, dadurch gekennzeichnet, daß die Differenzationsproteine von einem Fötus mit einem Alter von drei bis vier Monaten stammen.

12. Zusammensetzung zur diagnostischen Verwendung, enthaltend als aktives Element wenigstens ein Differenziationsprotein oder einen monoclonalen Antikörper nach einem der Ansprüche 1 oder 9 und ein passendes Substrat.

13. Zusammensetzung nach Anspruch 12, dadurch gekennzeichnet, daß das Differenziationsprotein oder der Antikörper markiert ist.

14. Zusammensetzung nach Anspruch 12 oder 13, dadurch gekennzeichnet, daß es sich um eine radioaktive, paramagnetische oder fluoreszierende Markierung handelt.

15. Zusammensetzung zur therapeutischen Verwendung, umfassend wenigstens ein Differenziationsprotein nach Anspuch 1 oder einen monoclonalen Antikörper nach Anspruch 9 in Verbindung mit einem aktiven Inhaltsstoff.

## Claims

1. Acino-foetal differentiation proteins associated with cancer of the pancreas, characterised in that the substances are manoside glycoproteins having an average apparent molecular weight chosen from between: 120 KD, 94 KD, 75 KD and 58 KD and coming inter alia from human foetal pancreas aged less than 6 months'gestation.

2. A method of preparing proteins according to claim 1, characterised in that a homogenate of soluble human foetal pancreas is prepared and a fraction made up of manoside glycoproteins is isolated.

3. A method of preparing acino-foetal differentiation proteins associated with cancer of the pancreas from human foetal pancreatic extracts according to claim 2 and comprising the following steps:

a) A homogenate of soluble human foetal pancreas is prepared,

b) The homogenate is treated by af finity column chromatography on concanavalin A coupled to a gel in order to fix the proteins,

c) The proteins in question are eluted and

d) The resulting proteins are purified if necessary.

4. A method according to claim 2 or 3, characterised in that the human foetal pancreas is aged less than 6 months, preferably less than 5 months.

5. A polyclonal antiserum agatnst proteins according to claim 1, characterised in that it is specific to cancer of the pancreas.

6. A method of preparing an antiserum according to claim 5, comprising the following steps:

a) An extract of human foetal pancreas less than 6 months old is injected into an animal,

b) The animal is bled and an antiserum is recovered,

c) The antiserum is decomplemented,

d) The decomplemented antiserum is absorbed,

e) The adsorbed decomplemented antiserum is centrifuged, and

f) The specific antiserum is recoverd.

7. A method of preparing an antiserum according to clalm 5, characterised in that an extract of human foetal pancreas from a foetus aged less than 5 months is injected.

8. A method of preparing an antiserum according to claim 6 or 7, characterised In that the antiserum is absorbed, more particularly on A, O or B erythrocytes, normal serum and/or normal pancreas.

9. A monoclonal antibody specific to a protein according to claim 1.

10. A method of preparing a monoclonal antibody specific to a protein according to claim 1, characterised in that it comprises the following steps:

a) A mouse is immunised with at least one of the differentiation proteins,

b) Cellular fusion is brought about between spleen cells of the mice and an SP$_2$ myeloma,

c) The resulting hybridomas are cultivated in a suitable medium,

d) The cultures are selected by sieving,

e) The positive hybridomas after sieving are conserved and cloned specifically with the foetal pancreas, and

f) The monoclonal antibody is isolated and purified if necessary.

11. A method according to claim 10, characterised in that the differentiation proteins come from a foetus aged 3 to 4 months.

12. A composition for diagnostic use in which the active constituent is at least one differentiation protein or monoclonal antibody according to claim 1 or 9 and an acceptable substrate.

13. A composition according to claim 12, characterised in that the differentiation protetn or the antibody is tagged.

14. A composition according to claim 12 or 13, characterised in that the tagging is radioactive, paramagnetic or fluorescent.

15. A composition for therapeutic use containing at least one differentiation protein according to claim 1 or one monoclonal antibody according to claim 9 conjugated with an active principle.